# EUROPÄISCHE PATENTSCHRIFT

(11) **EP 2 822 609 B1**
(45) Veröffentlichungstag und Bekanntmachung des Hinweises auf die Patenterteilung: **20.01.2016**
(21) Anmeldenummer: 13707140.3
(22) Anmeldetag: 22.02.2013
(51) Int. Cl.: A61L 2/26, A61B 19/02

(54) **CHIRURGISCHER STERILISIERBEHÄLTER MIT FLUIDEXTRAKTIONSVORRICHTUNG**
SURGICAL STERILISATION CONTAINER WITH FLUID EXTRACTION DEVICE
RÉCIPIENT DE STÉRILISATION CHIRURGICAL COMPRENANT UN DISPOSITIF D'EXTRACTION DE FLUIDES

(30) Priorität: 05.03.2012 DE 102012101832
(43) Veröffentlichungstag der Anmeldung: 14.01.2015
(73) Patentinhaber: Aesculap AG, 78532 Tuttlingen (DE)
(72) Erfinder: GRAY-DREIZLER, John, 78628 Rottweil (DE); WEISSHAUPT, Dieter, 78194 Immendingen (DE); SCHUSTER, Stefan, 78048 Villingen-Schwenningen (DE)
(74) Vertreter: Hoeger, Stellrecht & Partner Patentanwälte mbB
(86) Internationale Anmeldenummer: PCT/EP2013/053530
(87) Internationale Veröffentlichungsnummer: WO 2013/131760

(56) Entgegenhaltungen:
- EP-A1- 1 647 285
- WO-A2-2008/061137
- DE-A1- 19 827 442
- US-A- 2 673 379

## Beschreibung

Die Erfindung betrifft einen chirurgischen Sterilisierbehälter, umfassend einen Boden und eine Behälterwand, wobei am Sterilisierbehälter eine Durchgangsöffnung zum Medienaustausch gebildet ist, sowie umfassend eine Ventileinrichtung, die ein Auslassventil zum Freigeben und Verschließen der Durchgangsöffnung aufweist.

Es sind chirurgische Sterilisierbehälter der eingangs genannten Art bekannt, in denen chirurgische Instrumente zum Sterilisieren aufnehmbar sind. Während des Sterilisiervorgangs bildet sich Fluid, insbesondere Kondensat, in einem vom Sterilisierbehälter definierten Behälterinnenraum. Das Kondensat kann beispielsweise während einer Trocknungsphase nach dem Sterilisiervorgang verdampft werden. Über eine am Sterilisierbehälter gebildete Durchgangsöffnung, die mittels des Auslassventils freigebbar und verschließbar ist, kann der Dampf aus dem Behälterinnenraum austreten.

Da sich Kondensat üblicherweise am Boden des Sterilisierbehälters sammelt, wurden Sterilisierbehälter entwickelt, bei denen die Durchgangsöffnung im Boden gebildet ist und das Auslassventil die Durchgangsöffnung im Boden freigeben und verschließen kann. Dies dient dazu, das am Boden gesammelte Kondensat nach außen aus dem Sterilisierbehälter abzulassen. Das Ablassen von Kondensat birgt, insbesondere bei Einsatz eines druckbetätigbaren Auslassventils, allerdings den großen Nachteil in sich, dass abgelassenes Kondensat schwallartig aus dem Sterilisierbehälter austritt. Im Falle übereinander gestapelter Sterilisierbehälter kann das Kondensat vom höher gelegenen Sterilisierbehälter über den Deckel des darunter gelegenen Sterilisierbehälters fließen. Dies führt zu einer unerwünschten Abkühlung des tiefer gelegenen Sterilisierbehälters und unerwünschter Kondensatnachbildung in dessen Behälterinnenraum, welches zusätzlich verdampft oder abgelassen werden muss. Als besonders nachteilig erweist sich die Durchgangsöffnung im Boden auch deswegen, weil der Sterilisierbehälter bei einer Fehlfunktion oder Beschädigung des Auslassventils unzureichend abgedichtet ist. Dies kann zu einem unzureichenden Sterilisierergebnis führen und das Eintreten von Keimen in den Behälterinnenraum begünstigen. Speziell bei Anordnung der Durchgangsöffnung im und des Auslassventils am Boden besteht ein hohes Risiko, dass Unebenheiten einer Aufstellfläche für den Sterilisierbehälter oder auf der Aufstellfläche vorhandene Gegenstände von unten auf das Auslassventil einwirken und zu dessen Beschädigung oder Fehlfunktion führen. Eine unebene Aufstellfläche oder Gegenstände auf der Aufstellfläche können weiter dazu führen, dass der Ventilkörper vom Ventilsitz des Auslassventils angehoben und die Durchgangsöffnung freigegeben wird, so dass selbst bei an sich intaktem Auslassventil eine große Gefahr des Eintritts von Keimen in den Behälterinnenraum besteht. Weiter erweist sich als nachteilig, dass aufgrund der Anordnung der Durchgangsöffnung im Boden eine Fehlfunktion oder Beschädigung des Auslassventils für üblicherweise mit dem Sterilisierbehälter hantierendes Krankenhauspersonal schwer zu erkennen ist.

Sterilisierbehälter der vorstehend beschriebenen Art mit einer Durchgangsöffnung im Boden und am Boden angeordnetem Auslassventil sind beispielsweise in der US 4,900,519 und der EP 1 035 873 B1 beschrieben.

Weitere Sterilisierbehälter sind in der WO 2008/061137 A2, der EP 1 647 285 A1, der US 2,673,379 und der DE 198 27 442 A1 beschrieben.

"Aufstellfläche" ist eine Fläche, auf welcher der Sterilisierbehälter positionierbar ist. "Aufstellebene" ist eine vom Sterilisierbehälter definierte Kontaktebene, in welcher der Sterilisierbehälter die Aufstellfläche kontaktiert. Bei üblicherweise horizontal ausgerichteter Aufstellfläche führt bei bestimmungsgemäßem Gebrauch des Sterilisierbehälters in einer Betriebsstellung zu einer horizontalen Ausrichtung der Aufstell- oder Kontaktebene. Positions- und Orientierungsangaben wie beispielsweise "oben", "unten" beziehen sich vorliegend auf eine Betriebsstellung des Sterilisierbehälters, in der dieser bestimmungsgemäß auf einer Aufstellfläche positioniert ist.

Aufgabe der vorliegenden Erfindung ist es, einen chirurgischen Sterilisierbehälter der eingangs genannten Art bereitzustellen, aus dem im Behälterinnenraum gebildetes Fluid unter Verringerung der Gefahr eines Eintritts von Keimen in den Behälterinnenraum entfernbar ist.

Diese Aufgabe wird erfindungsgemäß durch einen Sterilisierbehälter mit den Merkmalen von Anspruch 1 gelöst.

Bei dem erfindungsgemäßen Sterilisierbehälter ist das Auslassventil im Abstand zum Boden angeordnet. Die Gefahr, dass eine unebene Aufstellfläche oder auf der Aufstellfläche vorhandene Gegenstände, wie bei dem vorstehend beschriebenen Sterilisierbehälter, auf das Auslassventil einwirken und dieses unerwünschterweise öffnen oder beschädigen, kann dadurch weitestgehend vermieden werden. Dies ermöglicht eine zuverlässige Funktion des Auslassventils zum Abdichten der Durchgangsöffnung in der Behälterwand, um das Eintreten von Keimen in den Behälterinnenraum, auch nach dem Ende des Sterilisiervorgangs, zu vermeiden. Zum Entfernen von Fluid aus dem Behälterinnenraum, und dadurch zur Verkürzung der Trocknungsphase, weist der erfindungsgemäße Sterilisierbehälter eine Fluidhebeeinrichtung auf. Die Fluidhebeeinrichtung bildet eine Fluidverbindung vom Boden aus, an dem sich Fluid wie insbesondere Kondensat üblicherweise sammelt, so dass Fluid mittels der Fluidhebeeinrichtung angehoben und dem Auslassventil zugeführt werden kann. Fluid kann dadurch durch die Behälterwand insbesondere seitlich aus dem Sterilisierbehälter austreten. Dies erlaubt es, den vorstehend erwähnten Nachteil des bekannten Sterilisierbehälters, bei der Fluid schwallartig nach unten aus dem Sterilisierbehälter austritt, zu vermeiden. Beim erfindungsgemäßen Sterilisierbehälter kann insbesondere vorgesehen sein, dass zusätzlich zum Anheben von Fluid mittels der Fluidhebeeinrichtung und Zuführen zum Auslassventil der Behälterinnendruck, über einen Nebenstrompfad an der Fluidhebeeinrichtung vorbei, abgebaut werden kann. Dies gibt auch die Möglichkeit, die mechanische Belastung auf den Sterilisierbehälter zu verringern.

Von Vorteil ist es, dass die Fluidhebeeinrichtung einen Fluidkanal umfasst zum Bereitstellen der Fluidverbindung, durch den hindurch Fluid vom Boden zum Auslassventil anhebbar ist und der eine Kanaleintrittsöffnung für Fluid umfasst sowie eine im Abstand dazu angeordnete Kanalaustrittsöffnung für Fluid. Durch die Kanaleintrittsöffnung, die vorzugsweise am oder nahe dem Boden angeordnet ist, kann Fluid in den Fluidkanal eintreten. Fluid kann durch den Fluidkanal hindurch strömen und durch die Austrittsöffnung hindurch aus dem Fluidkanal austreten. Die Fluidströmung kann insbesondere dadurch hervorgerufen werden, dass bei geöffnetem Auslassventil infolge der Druckdifferenz zwischen dem Umgebungsdruck und dem Druck im Behälterinnenraum eine Saugströmung im Fluidkanal vorliegt. Fluid kann dadurch in den Fluidkanal eingesaugt, angehoben und dem Auslassventil zugeführt werden.

Günstig ist es, dass der Fluidkanal mit einem die Kanaleintrittsöffnung bildenden Ende in eine im Boden gebildete Vertiefung eingreift, die einen Fluidsammelbereich zum Sammeln von Fluid ausbildet. Im Fluidsammelbereich kann Fluid wie insbesondere Kondensat gesammelt werden. Dadurch, dass der Fluidkanal in den Fluidsammelbereich eingreift, kann eine wirkungsvolle Fluidverbindung vom Fluidsammelbereich zum Auslassventil zum Anheben des Fluids bereitgestellt werden.

Vorzugsweise ist der Boden frei von Durchgangsöffnungen, so dass der Boden eine Sterilbarriere des Sterilisierbehälters bilden kann. Die bei dem vorstehend erwähnten herkömmlichen Sterilisierbehälter auftretenden Nachteile aufgrund der Durchgangsöffnung im Boden können dadurch vermieden werden. Günstig ist es, wenn der Sterilisierbehälter eine den Boden umfassende Sterilisierbehälterwanne umfasst, die eine vom Boden abstehende Außenwand aufweist. Die Außenwand bildet eine Behälterwand des Sterilisierbehälters aus. Die Behälterwand kann auch eine von einem Sterilisierbehälterdeckel gebildete Deckenwand umfassen.

Zur Erzielung einer konstruktiv einfachen Ausgestaltung des Sterilisierbehälters ist es günstig, wenn die Durchgangsöffnung in der Außenwand gebildet ist.

In der Behälterwand kann mindestens eine weitere Durchgangsöffnung gebildet sein, beispielsweise eine Durchgangsöffnung, die mittels eines Einlassventils der Ventileinrichtung freigebbar und verschließbar ist. In einer weiteren Durchgangsöffnung kann ein Filter des Sterilisierbehälters angeordnet sein, durch den hindurch der Medienaustausch zwischen dem Behälterinnenraum und der Umgebung erfolgen kann, wenn die Druckdifferenzen hinreichend gering sind, so dass weder das Auslassventil noch das Einlassventil öffnen. Die

Durchgangsöffnungen, in denen das Einlassventil bzw. der Filter angeordnet sind, sind vorzugsweise in der Außenwand gebildet.

Es kann vorgesehen sein, dass die Sterilisierbehälterwanne im Querschnitt rechteckförmig oder im Wesentlichen rechteckförmig ausgebildet ist mit vier die Außenwand bildenden Seitenwänden und dass die Durchgangsöffnung in einer Seitenwand gebildet ist.

Als vorteilhaft erweist es sich in der Praxis, wenn die Außenwand Längsseitenwände und Querseitenwände umfasst und wenn die Durchgangsöffnung in einer Querseitenwand gebildet ist. Beispielsweise ist neben der Durchgangsöffnung in der Querseitenwand eine weitere Durchgangsöffnung gebildet, in der ein Einlassventil angeordnet ist. In einer der Querseitenwand gegenüberliegenden Querseitenwand kann eine Durchgangsöffnung gebildet sein, in der ein Filter angeordnet ist.

Wie bereits erwähnt, kann der Sterilisierbehälter einen Sterilisierbehälterdeckel aufweisen. Der Deckel kann lösbar auf eine Sterilisierbehälterwanne des Sterilisierbehälters aufsetzbar sein.

Bei einer konstruktiv einfachen Ausgestaltung und zuverlässigen Funktion ist das Auslassventil als Überdruckventil ausgebildet, das bei Vorliegen einer vorgegebenen oder vorgebbaren Druckdifferenz zwischen dem Behälterinnenraum und der Umgebung öffnet und die Durchgangsöffnung freigibt. Durch das Auslassventil kann über Fluid hinaus im Behälterinnenraum vorhandenes Gas austreten.

Ebenfalls zur Erzielung einer konstruktiv einfachen Ausgestaltung ist es günstig, wenn die Fluidhebeeinrichtung druckbetätigbar ist und Fluid beispielsweise vom Boden abhängig von der Druckdifferenz zwischen dem Behälterinnenraum und der Umgebung des Sterilisierbehälters anhebt und dem Auslassventil zuführt.

Vorzugsweise ist die Kanaleintrittsöffnung in Richtung auf den Boden gerichtet. Darunter kann insbesondere verstanden werden, dass eine der Durchtrittsrichtung von Fluid in den Fluidkanal entgegengesetzte Richtung in Richtung des Bodens weist. Das Anheben von Fluid kann dadurch vereinfacht werden.

Vorteilhafterweise weist die Kanaleintrittsöffnung eine Neigung relativ zum Boden auf. Darunter kann insbesondere verstanden werden, dass ein die Eintrittsöffnung einfassender Rand des Fluidkanals relativ zum Boden geneigt ist. Beispielsweise weist der Fluidkanal an seinem dem Boden zugewandten Ende eine Abschrägung auf. Bei vorgegebenem Fluidkanalquerschnitt kann dadurch eine vergrößerte Fluideintrittsöffnung ausgebildet werden, die das Anheben von Fluid durch Absaugen durch den Fluidkanal hindurch erleichtert.

Das die Fluideintrittsöffnung ausbildende Ende des Fluidkanals weist günstigerweise einen Abstand vom Boden auf, um das Ansaugen von Fluid in den Fluidkanal zu erleichtern.

Der Fluidsammelbereich ist beispielsweise an einer Seitenwand der Sterilisierbehälterwanne angeordnet, beispielsweise an einer Querseitenwand und insbesondere an einem Eckbereich des Bodens, an dem eine Querseitenwand und eine Längsseitenwand der Sterilisierbehälterwanne aufeinander treffen.

Vorzugsweise ist die Kanalaustrittsöffnung auf einen Ventilkörper des Auslassventils gerichtet. Darunter kann insbesondere verstanden werden, dass die Durchtrittsrichtung von Fluid durch die Kanalaustrittsöffnung auf den Ventilkörper weist, um Fluid auf wirkungsvolle Weise dem Ventilkörper zuzuführen.

Für eine konstruktiv einfache Ausgestaltung ist es von Vorteil, wenn die Kanalaustrittsöffnung auf eine Aufstromseite des Auslassventils gerichtet ist, speziell auf die Aufstromseite des Ventilkörpers.

Günstig ist es, wenn die Kanalaustrittsöffnung an einer dem Boden zugewandten Seite des Auslassventils angeordnet ist, um Fluid vom Boden um eine möglichst geringe Strecke anzuheben.

Von Vorteil ist es, wenn die Fluidhebeeinrichtung einen Injektor zum Anheben von Fluid durch den Fluidkanal umfasst und wenn der Injektor eine Injektoreintrittsöffnung und eine Injektoraustrittsöffnung aufweist, über die eine Strömungsverbindung von einem vom Sterilisierbehälter definierten Behälterinnenraum zum Auslassventil bereitgestellt wird, sowie eine in Strömungsrichtung zwischen der Injektoreintrittsöffnung und der Injektoraustrittsöffnung angeordnete und vom Fluidkanal ausgebildete Injektorsaugöffnung. Bei der Fluidhebeeinrichtung dieser vorteilhaften Ausführungsform ist ein Injektor vorgesehen. Durch den Injektor hindurch, von einer Injektoreintrittsöffnung bis zu einer Injektoraustrittsöffnung, kann eine Strömungsverbindung vom Behälterinnenraum zum Auslassventil bereitgestellt werden. Infolge einer Druckdifferenz zwischen dem Behälterinnenraum und der Umgebung des Sterilisierbehälters kann sich durch den Injektor hindurch bei geöffnetem Auslassventil eine wirkungsvolle Saugströmung ausbilden. Zwischen der Injektoreintrittsöffnung und der Injektoraustrittsöffnung weist der Injektor eine Injektorsaugöffnung auf, die vom Fluidkanal gebildet wird. Durch die sich ausbildende Saugströmung kann eine Druckdifferenz am Fluidkanal entstehen zwischen der Injektorsaugöffnung und der in Strömungsrichtung des Fluids durch den Fluidkanal vorgelagerten Kanaleintrittsöffnung. Dies erlaubt es, Fluid durch den Fluidkanal hindurch anzuheben und dem Auslassventil zuzuführen. Es zeigt sich in der Praxis, dass Fluid auf diese Weise wirkungsvoll angehoben und aus dem Behälter entfernt werden kann.

Zur konstruktiven Vereinfachung ist es günstig, wenn der Injektor in den Fluidkanal integriert ist, wobei vorzugsweise die Injektoraustrittsöffnung durch die Kanalaustrittsöffnung gebildet ist und/oder die Injektoreintrittsöffnung in einer Kanalwand des Fluidkanals gebildet ist. Die Injektoreintrittsöffnung kann beispielsweise eine Eintrittsöffnung der Kanalwand sein, durch die hindurch ein Nebenstrompfad vom Behälterinnenraum zum Auslassventil gebildet ist unter Umgehung des Fluidkanals von der Kanaleintrittsöffnung zur Injektorsaugöffnung.

Der Injektor kann, zum Beispiel der Saugöffnung in Strömungsrichtung nachgelagert, einen Diffusor umfassen, um die Strömung angesaugten und angehobenen Fluids zu beruhigen. Der Diffusor kann in den Fluidkanal integriert sein.

Von Vorteil ist es, insbesondere bei Vorhandensein eines Injektors der Fluidhebeeinrichtung, wenn der Fluidkanal eine Querschnittsverengung aufweist. Dies begünstigt die Ausbildung einer Druckdifferenz im Fluidkanal, um Fluid vom Boden zum Auslassventil anzuheben. Der Querschnittsverengung nachgelagert kann sich der Fluidkanalquerschnitt zur Ausbildung eines Diffusors erweitern.

Von Vorteil ist es, wenn, bezogen auf die Strömungsrichtung des vom Boden zum Auslassventil strömenden Fluids, der Querschnittsverengung vorgelagert eine Eintrittsöffnung im Fluidkanal gebildet ist, über die eine Strömungsverbindung von einem vom Sterilisierbehälter definierten Behälterinnenraum zum Auslassventil bereitgestellt wird. Bei der Eintrittsöffnung handelt es sich insbesondere um die vorstehend erwähnte Injektoreintrittsöffnung des Injektors der Fluidhebeeinrichtung.

Bei einer vorteilhaften Ausführungsform des erfindungsgemäßen Sterilisierbehälters ist vorgesehen, dass der Fluidkanal einen ersten, in einer vom Boden weg weisenden Richtung ausgerichteten Kanalabschnitt aufweist, sowie einen, bezogen auf die Strömungsrichtung des vom Boden zum Auslassventil strömenden Fluids, dem ersten Kanalabschnitt nachgelagerten zweiten Kanalabschnitt, der einen Winkel mit dem ersten Kanalabschnitt einschließt. Dadurch kann beispielsweise die Gefahr verringert werden, dass angehobenes Fluid "sprühstoßartig" aus dem Sterilisierbehälter austritt.

Der erste Kanalabschnitt ist günstigerweise rechtwinklig oder im Wesentlichen rechtwinklig zu einer vom Sterilisierbehälter definierten Aufstellebene ausgerichtet und kann dadurch vom Boden insbesondere senkrecht nach oben weisen.

Als vorteilhaft erweist es sich, wenn der zweite Kanalabschnitt eine Neigung relativ zu einer vom Sterilisierbehälter definierten Aufstellebene aufweist. Darunter kann insbesondere verstanden werden, dass der zweite Kanalabschnitt in Richtung auf die Aufstellebene geneigt ist. Durch den ersten Kanalabschnitt angehobenes Fluid kann im Fluidkanal durch den zweiten Kanalabschnitt hindurch wieder in Richtung der Aufstellebene strömen, bevor es zum Auslassventil gelangt. Die Fluidströmung kann sich dadurch beruhigen, so dass Fluid nicht sprühstoßartig aus dem Sterilisierbehälter austritt.

Beispielsweise kann vorgesehen sein, dass der erste Kanalabschnitt und der zweite Kanalabschnitt einen Winkel kleiner als 90° miteinander einschließen, beispielsweise indem der erste Kanalabschnitt senkrecht zur Aufstellebene ausgerichtet ist und der zweite Kanalabschnitt auf diese gerichtet ist.

Zur Erzielung einer konstruktiv einfachen und kompakten Ausgestaltung erweist es sich als vorteilhaft, wenn der Fluidkanal abschnittsweise von einer Kanalwand begrenzt ist, die von einem Ventilhalteteil gebildet ist, an welchem das Auslassventil gehalten ist und das an der Behälterwand festgelegt ist. Das Ventilhalteteil bildet zumindest teilweise die Kanalwand des Fluidkanals aus. Zu diesem Zweck kann das Ventilhalteteil beispielsweise eine nut- oder rillenförmige Vertiefung aufweisen.

Vorzugsweise umfasst die Fluidhebeeinrichtung eine den Fluidkanal zumindest abschnittsweise begrenzende Kanalwand, die eine das Auslassventil behälterinnenseitig überdeckende Abdeckung ausbildet. Auch dies ermöglicht eine konstruktive Vereinfachung, bei der eine Kanalwand zugleich zum Abdecken des Auslassventils eingesetzt werden kann.

Als günstig erweist es sich, wenn in der Abdeckung mindestens eine Öffnung zum Ausbilden einer Strömungsverbindung von einem vom Sterilisierbehälter definierten Behälterinnenraum zum Auslassventil vorgesehen ist. Dies ermöglicht es, den Druck im Behälterinnenraum unter Ausbildung eines Nebenstrompfades und unter Umgehung des Fluidkanals abzubauen. Gas kann durch die mindestens eine Öffnung hindurch zum geöffneten Auslassventil strömen und aus dem Sterilisierbehälter austreten.

Es kann vorgesehen sein, dass das Auslassventil einen Ventilkörper aufweist, der in mindestens eine in der Abdeckung gebildete Öffnung eingreift. Die Öffnung kann dadurch als Ausrichtelement für den Ventilkörper wirken. Beispielsweise weist der Ventilkörper einen zapfenartigen Vorsprung auf, der in die Öffnung eingreift.

Zur Erzielung einer konstruktiv einfachen Ausgestaltung ist es von Vorteil, wenn die Abdeckung mit dem Ventilhalteteil verbunden ist und der Fluidkanal zwischen der Abdeckung und dem Ventilhalteteil gebildet ist. Zu diesem Zweck bildet das Ventilhalteteil bevorzugt mindestens eine Kanalwand des Fluidkanals aus. Beispielsweise ist im Ventilhalteteil eine nut- oder rillenförmige Vertiefung vorgesehen, die von der Abdeckung abgedeckt ist.

Günstig ist es, wenn der Fluidkanal formstabil ausgebildet ist. Dies ermöglicht eine robuste Konstruktion des Fluidkanals, der sich insbesondere auch bei hohen Drücken im Behälterinnenraum nicht verformt. Dies begünstigt auch eine zuverlässige Funktion der Fluidhebeeinrichtung.

Der Fluidkanal kann zur Erzielung einer kompakten Bauform beispielsweise an einer Seitenwand des Sterilisierbehälters festgelegt sein, wobei er mittelbar oder unmittelbar an der Seitenwand festgelegt sein kann. Beispielsweise ist der Fluidkanal am Ventilhalteteil festgelegt oder von diesem teilweise ausgebildet, welches an einer Seitenwand und insbesondere einer Querseitenwand des Sterilisierbehälters festgelegt ist.

Zur Erzielung einer kompakten Bauform verläuft der Fluidkanal vorzugsweise parallel zu einer Seitenwand und insbesondere einer Querseitenwand des Sterilisierbehälters.

Die Fluidhebeeinrichtung kann beispielsweise ganz oder teilweise seitlich neben dem Auslassventil angeordnet sein, ebenfalls um eine kompakte Bauform zu erzielen.

Zur Erzielung des gleichen Vorteils kann vorgesehen sein, dass die Fluidhebeeinrichtung ganz oder teilweise unter dem Auslassventil angeordnet ist. Beispielsweise ist die Fluidhebeeinrichtung zwischen dem Boden und dem Auslassventil angeordnet.

Wie bereits erwähnt, kann der Sterilisierbehälter ein Ventilhalteteil umfassen, an dem das Auslassventil gehalten ist und das an der Behälterwand festgelegt ist, beispielsweise an einer Querseitenwand des Sterilisierbehälters. Dadurch ist die Möglichkeit gegeben, je nach Sterilisierbehälter und/oder Anforderungen an den Sterilisiervorgangvorgang unterschiedliche Ventilhalteteile mit unterschiedlichen Auslassventilen und/oder daran angeordneten Fluidhebeeinrichtungen vorzusehen, die bedarfsgerecht mit der jeweiligen Behälterwand verbunden werden können.

Vorzugsweise ist das Ventilhalteteil lösbar an der Behälterwand festgelegt, so dass es bei Bedarf von dieser gelöst und ausgetauscht werden kann. Die Befestigung erfolgt zum Beispiel über Verrastung und/oder Verklemmung.

Vorteilhafterweise ist das Ventilhalteteil in die Durchgangsöffnung eingesetzt und bildet einen Ventilsitz des Auslassventils aus. Das Ventilhalteteil kann dichtend an der Behälterwand anliegen und in die Durchgangsöffnung in der Behälterwand eingreifen. Der Ventilsitz des Auslassventils kann zur Erzielung einer konstruktiven Vereinfachung durch das Ventilhalteteil ausgebildet werden. Die in der Behälterwand gebildete Durchgangsöffnung, soweit nicht durch das Ventilhalteteil selber abgedeckt, kann mittels des Auslassventils geschlossen und freigegeben werden.

Vorzugsweise ist das Ventilhalteteil plattenförmig oder im Wesentlichen plattenförmig, um eine konstruktiv einfache Ausgestaltung zu erzielen.

Wie bereits erwähnt, kann der Sterilisierbehälter ein Einlassventil umfassen. Beispielsweise kann in der Behälterwand eine weitere Durchgangsöffnung gebildet sein, wobei die Ventileinrichtung ein Einlassventil umfasst zum Freigeben und Verschließen der weiteren Durchgangsöffnung.

Zur Erzielung einer konstruktiv einfachen Ausgestaltung ist es günstig, wenn die Ventileinrichtung ein Einlassventil umfasst, das am Ventilhalteteil gehalten ist. Es kann auch vorgesehen sein, dass ein doppeltwirkendes Auslass- und Einlassventil vorgesehen ist.

Wie eingangs erwähnt, betrifft die Erfindung auch eine Fluidextraktionsvorrichtung zur Verwendung mit einem eine Behälterwand und einen Boden aufweisenden chirurgischen Sterilisierbehälter. Eine erfindungsgemäße Fluidextraktionsvorrichtung zum Extrahieren von Fluid aus dem Sterilisierbehälter wird durch die Merkmale von Anspruch 15 definiert.

Sich auf die Ventileinrichtung und die Fluidhebeeinrichtung beziehende Merkmale des erfindungsgemäßen Sterilisierbehälters sowie vorteilhafte Ausführungsformen desselben können bei der Ventileinrichtung und der Fluidhebeeinrichtung der erfindungsgemäßen Fluidextraktionsvorrichtung ebenfalls vorgesehen sein. Diesbezüglich kann auf voranstehende Erläuterungen verwiesen werden, in der diese Merkmale jeweils einschließlich der mit ihnen erzielbaren Vorteile genannt wurden. Diese Merkmale können zur Ausbildung vorteilhafter Ausführungsformen der erfindungsgemäßen Fluidextraktionsvorrichtung herangezogen werden. Insbesondere kann die Fluidhebeeinrichtung der erfindungsgemäßen Fluidextraktionsvorrichtung den vorstehend erwähnten Fluidkanal sowie den vorstehend erwähnten Injektor einschließlich ihrer jeweiligen Merkmale umfassen, wobei der Injektor vorzugsweise in den Fluidkanal integriert ist. Der Injektor kann den Diffusor umfassen, der in den Fluidkanal integriert sein kann. Weiter ist günstigerweise vorgesehen, dass die Fluidextraktionsvorrichtung ein vorzugsweise einstückiges Ventilhalteteil umfasst, an dem das Auslassventil gehalten ist und das an der Behälterwand vorzugsweise lösbar festlegbar ist, und dass die Fluidhebeeinrichtung am Ventilhalteteil angeordnet ist, beispielsweise mit diesem verbunden, mit diesem verbindbar oder zum Teil von diesem ausgebildet ist. Beispielsweise kann eine Kanalwand des Fluidkanals vom Ventilhalteteil gebildet werden. Eine Kanalwand des Fluidkanals kann eine Abdeckung des Auslassventils bilden. Der Fluidkanal kann zwischen dem Ventilhalteteil und der Abdeckung gebildet sein. Der Fluidkanal kann im Winkel, zum Beispiel kleiner als 90°, zueinander ausgerichtete Kanalabschnitte umfassen. Das Ventilhalteteil kann den Ventilsitz des Auslassventils ausbilden und/oder falls vorhanden, des Einlassventils.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung eines erfindungsgemäßen Sterilisierbehälters im geöffneten Zustand, umfassend eine erfindungsgemäße Fluidextraktionsvorrichtung;
- Figur 2:: eine perspektivische Darstellung der Fluidextraktionsvorrichtung des Sterilisierbehälters aus Figur 1;
- Figur 3:: eine Schnittansicht längs der Linie 3-3 in Figur 1;
- Figur 4:: eine Draufsicht auf eine Sterilisierbehälterwanne des Sterilisierbehälters aus Figur 1;
- Figur 5:: eine Schnittansicht längs der Linie 5-5 in Figur 4;

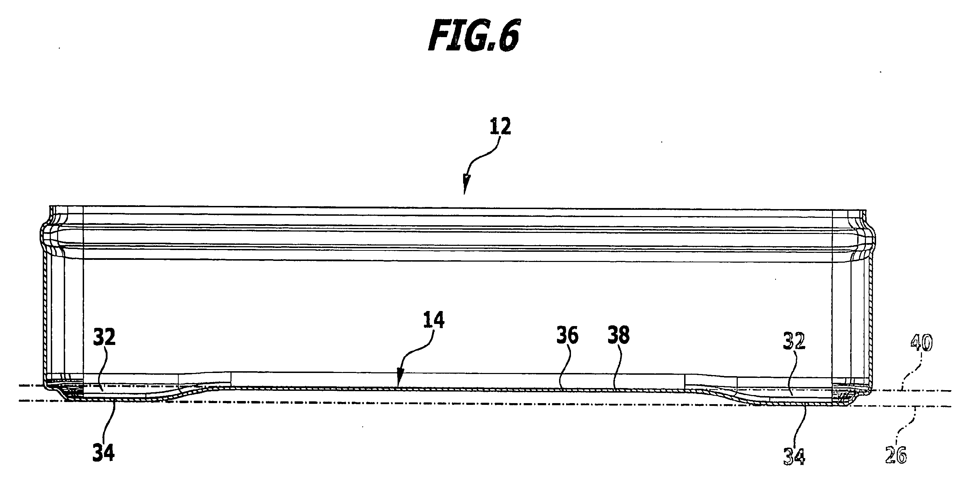

- Figur 6:: eine Schnittansicht längs der Linie 6-6 in Figur 4 und
- Figur 7:: eine perspektivische Darstellung einer Sterilisierbehälterwanne des Sterilisierbehälters aus Figur 1.

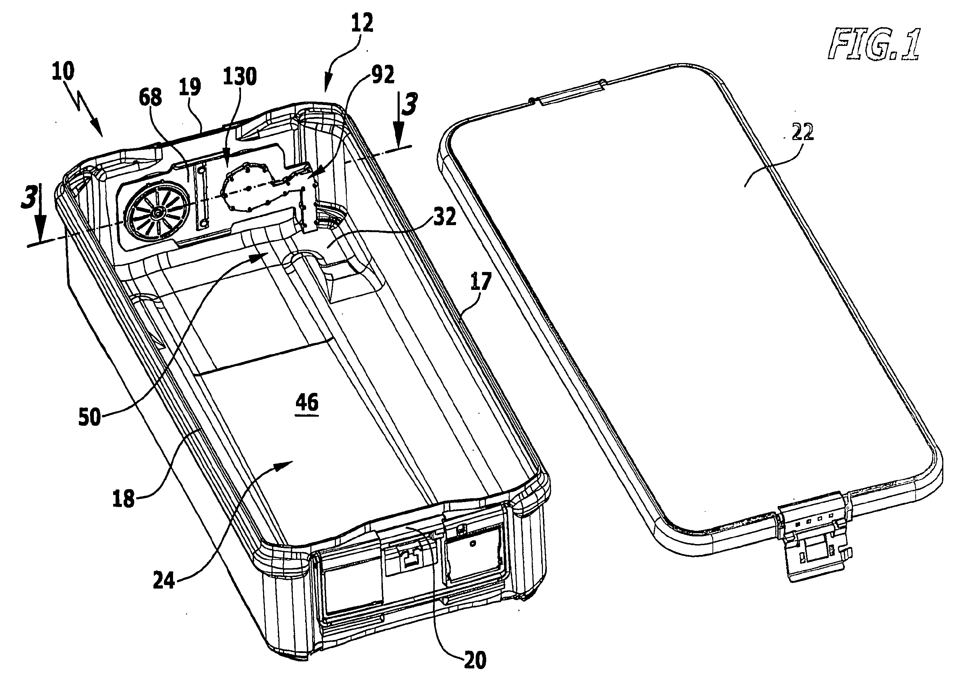

Figur 1 zeigt in perspektivischer Darstellung eine insgesamt mit dem Bezugszeichen 10 belegte bevorzugte Ausführungsform eines erfindungsgemäßen Sterilisierbehälters. Der Sterilisierbehälter 10 dient zur Aufnahme von chirurgischen Instrumenten während des Sterilisiervorgangs. Die in der Zeichnung nicht dargestellten Instrumente sind üblicherweise in einer in der Zeichnung ebenfalls nicht dargestellten Aufnahme angeordnet, beispielsweise in einem chirurgischen Siebkorb, der im Sterilisierbehälter 10 aufgenommen ist.

Der Sterilisierbehälter 10 umfasst eine Sterilisierbehälterwanne 12 von im Wesentlichen rechteckförmiger Gestalt, die einen Boden 14 umfasst sowie eine vom Boden 14 abstehende Außenwand 16. Der Boden 14 wird gebildet durch eine Bodenwand 15. Die Außenwand 16 umfasst vier Seitenwände, nämlich zwei Längsseitenwände 17 und 18, die endseitig mittels zweier Querseitenwände 19 bzw. 20 miteinander verbunden sind. Die Außenwand 16 ist eine Behälterwand des Sterilisierbehälters 10. Die Längsseitenwände 17 und 18 definieren eine Längsrichtung des Sterilisierbehälters 10, die Querseitenwände 19 und 20 definieren dessen Querrichtung.

Der Sterilisierbehälter 10 weist einen Sterilisierbehälterdeckel 22 auf, der dichtend auf die Sterilisierbehälterwanne 12 aufgesetzt werden kann, um diese abzudecken und einen vom Sterilisierbehälter 10 definierten Behälterinnenraum 24 zu verschließen. Mittels an sich bekannter Verschlusselemente kann der Sterilisierbehälterdeckel 22 mit der Sterilisierbehälterwanne 12 lösbar verbunden werden.

Die Sterilisierbehälterwanne 12 definiert eine Aufstellebene 26 des Sterilisierbehälters 10, bei der es sich um eine Kontaktebene handelt, in der die Sterilisierbehälterwanne 12 eine Aufstellfläche kontaktiert, auf welcher sie aufgestellt wird. Bei üblicherweise horizontal ausgerichteter Aufstellfläche ist die Aufstellebene 26 horizontal ausgerichtet.

Positions- und Orientierungsangaben wie beispielsweise "oben", "unten" oder dergleichen beziehen sich vorliegend auf eine Gebrauchsstellung des Sterilisierbehälters 10 auf einer horizontalen Aufstellfläche und damit horizontal ausgerichteter Aufstellebene 26 in einer Betriebsstellung des Sterilisierbehälters 10.

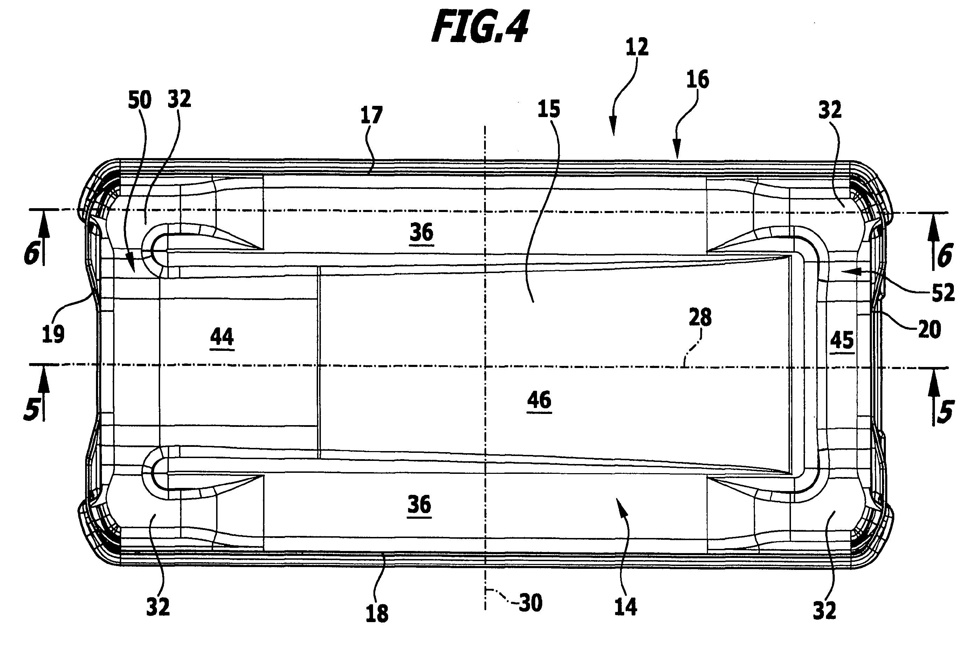

Die Sterilisierbehälterwanne 12, speziell der Boden 14, ist insgesamt symmetrisch ausgebildet bezüglich einer Symmetrieebene 28, die senkrecht zur Aufstellebene 26 ausgerichtet ist und eine Wannenmittenebene ist. Die Symmetrieebene 28 verläuft mittig zwischen den Längsseitenwänden 17 und 18, in Figur 4 senkrecht zur Zeichenebene und längs der Linie 5-5.

Weiter ist die Sterilisierbehälterwanne 12, speziell der Boden 14, asymmetrisch ausgebildet bezüglich einer Asymmetrieebene 30, die senkrecht zur Aufstellebene 26 und senkrecht zur Symmetrieebene 28 ausgerichtet ist und bei der es sich um eine Wannenmittenebene handelt, die mittig zwischen den Querseitenwänden 19 und 20 verläuft.

An Eckbereichen der Sterilisierbehälterwanne 12, an denen die Längsseitenwände 17, 18 und die Querseitenwände 19, 20 aufeinander stoßen, sind im Boden 14 Vertiefungen 32 vorhanden. Die Vertiefungen 32 werden, wie der Boden 14 im Übrigen, beim Formen der Sterilisierbehälterwanne 12 mittels eines Umformverfahrens gebildet, beispielsweise durch Tiefziehen. Durch Ausbilden der Vertiefungen 32 weist der Boden 14 außenseitig Aufstellelemente 34 auf, die die Aufstellebene 26 definieren.

Die Abschnitte der Bodenwand 15 im Bereich von in Längsrichtung einander gegenüberliegenden Vertiefungen 32 sind durch Bodenabschnitte 36 miteinander verbunden, die längs der Längsseitenwände 17 und 18 verlaufen. Die Bodenabschnitte 36 erstrecken sich in Längsrichtung über ungefähr 60 % der Länge und in Querrichtung über ungefähr 25 % des Bodens 14. Die Bodenabschnitte 36 sind planar ausgestaltet und bilden Tragelemente 38, die eine parallel zur Aufstellebene 26 ausgerichtete Tragebene 40 definieren. Über die Tragebene 40 ragen keine Abschnitte der Bodenwand 14 nach oben hinaus. Auf den Tragelementen 38 kann eine Aufnahme für chirurgische Instrumente, insbesondere ein chirurgischer Siebkorb, zuverlässig stehend positioniert werden.

In Querrichtung sind die Bodenabschnitte im Bereich der Vertiefungen 32 längs der Querseitenwand 19 mittels eines Bodenabschnittes 44 miteinander verbunden. Längs der Querseitenwand 20 sind die Bodenabschnitte im Bereich der Vertiefungen 32 mittels eines Bodenabschnittes 45 miteinander verbunden.

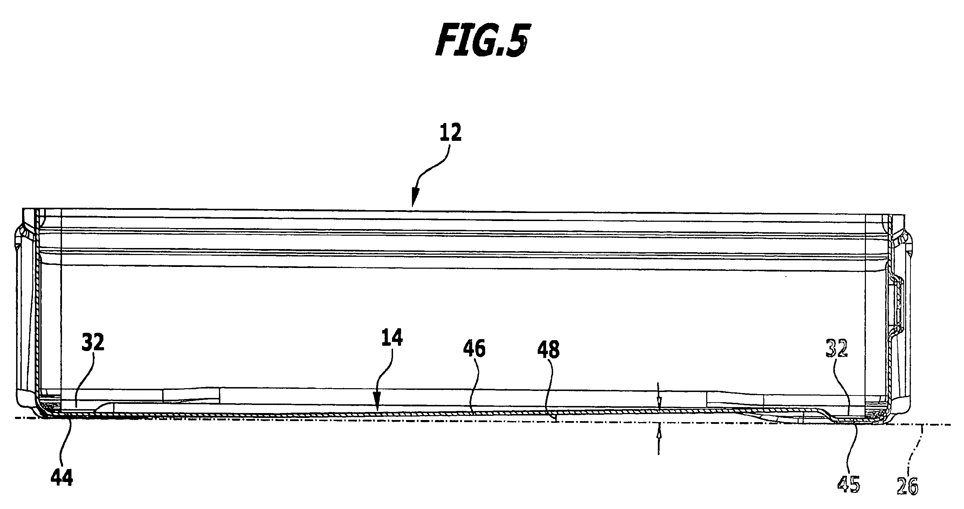

Die Bodenabschnitte 44 und 45 definieren eine gemeinsame Ebene, die parallel zur Aufstellebene 26 ausgerichtet ist und von dieser weniger weit beabstandet ist als von der Tragebene 40 (Figur 5). Der Bodenabschnitt 44 erstreckt sich in Längsrichtung über einen Bereich von ungefähr einem Viertel der Länge des Bodens 14, der Bodenabschnitt 45 über ungefähr 10% der Länge des Bodens 14. Im Abstand zur Querseitenwand 19 ist der Bodenabschnitt 44 mit einer Fluidableitfläche 46 des Bodens 14 verbunden, wobei die Verbindung in Querrichtung des Bodens 14 erfolgt.

Die Fluidableitfläche 46 erstreckt sich in Längsrichtung vom Bodenabschnitt 44 bis zum Bodenabschnitt 45 und in Querrichtung zwischen den Bodenabschnitten 36. Dabei überdeckt die Fluidableitfläche 46 die Mitte des Bodens 14, durch die die Symmetrieebene 28 und die Asymmetrieebene 30 verlaufen. Die Fluidableitfläche 46 ist planar ausgestaltet und in Draufsicht auf die Sterilisierbehälterwanne 12 ungefähr trapezförmig ausgebildet mit einer der Querseitenwand 20 zugewandten Basis. An der der Basis gegenüberliegenden Seite ist die Fluidableitfläche 46 mit dem Bodenabschnitt 44 verbunden. Insgesamt erstreckt sich die Fluidableitfläche 46 über ungefähr ein Drittel der Fläche des Bodens 14. Dabei überdeckt sie in Längsrichtung ungefähr 60 % bis ungefähr 70 % der Bodenfläche und in Querrichtung ungefähr 50 % der Bodenfläche.

Die Fluidableitfläche 46, insbesondere die von ihr definierte Ebene, ist relativ zur Aufstellebene 26 um einen Neigungswinkel 48 geneigt. Die Fluidableitfläche 46 schneidet die Aufstellebene nicht, da sie sich in Richtung auf die Aufstellebene nur bis zum Bodenabschnitt 44 erstreckt. Der Neigungswinkel 48 beträgt vorliegend weniger als 2°, beispielsweise ungefähr 1,5°. Die Fluidableitfläche 46 ist in Richtung auf die Querseitenwand 19 geneigt, so dass sie an ihrem der Querseitenwand 19 zugewandten Ende einen geringeren Abstand aufweist von der Aufstellebene 26 als an ihrem der Querseitenwand 19 gegenüberliegenden Ende. Letzteres Ende geht aus von der Tragebene 40, und das der Seitenwand 19 zugewandte Ende der Fluidableitfläche 46 ist in der vom Bodenabschnitt 44 definierten Ebene angeordnet.

Die Neigung der Fluidableitfläche 46 hat zur Folge, dass Fluid, insbesondere während des Sterilisiervorganges gebildetes Kondensat, von der Fluidableitfläche 46 in Richtung auf die Querseitenwand 19 abgeleitet wird. Da der Bodenabschnitt 44 und die Bodenwand 15 im Bereich der Vertiefungen 32 an der Querseitenwand 19 einen geringeren Abstand aufweisen von der Aufstellebene 26 als die Fluidableitfläche 46 (abgesehen von deren Verbindung mit dem Bodenabschnitt 44), wird Fluid dem Bodenabschnitt 44 und den Vertiefungen 32 an der Querseitenwand 19 zugeleitet. Dadurch bildet der Boden 14 im Bereich des Bodenabschnitts 44 und der Vertiefungen 32 an der Querseitenwand 19 einen Fluidsammelbereich 50 aus. Von der Fluidableitfläche 46 abgeleitetes Fluid sammelt sich im Fluidsammelbereich 50, wobei Fluid zunächst bis in die Vertiefungen 32 an der Querseitenwand 19 abgeleitet wird. Bei steigendem Fluidpegel kann sich Fluid auch auf dem Bodenabschnitt 44 sammeln, der bezüglich der Bodenwand 15 im Bereich der Vertiefungen 32 etwas erhöht liegt.

Bei vorstehend erwähnter Ausgestaltung verläuft der Fluidsammelbereich 50 entlang der Querseitenwand 19, die den Fluidsammelbereich 50 im Übergang zur Bodenwand 15 abschnittsweise begrenzt. Der Fluidsammelbereich 50 ist damit der Querseitenwand 19 benachbart. Auch von den Bodenabschnitten 36 kann Fluid in Längsrichtung über Abschrägungen der Bodenwand 15 in die Vertiefungen 32 an der Querseitenwand 19 geleitet werden.

Längs der Querseitenwand 20 weist der Boden 14 einen weiteren Fluidsammelbereich 52 auf. Der Fluidsammelbereich 52 wird gebildet durch die Vertiefungen 32 an der Querseitenwand 20 sowie den in Querrichtung dazwischen liegenden und vom Bodenabschnitt 45 untenseitig begrenzten Bereich. Von den Bodenabschnitten 36 kann Fluid auch in die Vertiefungen 32 an der Querseitenwand 20 abgeleitet werden. Insgesamt ist jedoch die Menge des sich im Fluidsammelbereich 52 sammelnden Fluids wesentlich geringer als die Menge des sich im Fluidsammelbereich 50 sammelnden Fluids. Dies ist auf die asymmetrische Ausgestaltung des Bodens 14 relativ zur Asymmetrieebene 30 und die geneigte Fluidableitfläche 46 zurückzuführen, mit der die überwiegende Anteil an Fluid in Richtung auf die Querseitenwand 19 in den Fluidsammelbereich 50 abgeleitet wird.

Um einen Medienaustausch von Medien wie Gas und/oder Fluid aus dem Behälterinnenraum 24 in die Umgebung und umgekehrt auch bei geschlossenem Sterilisierbehälter 10 zu ermöglichen, sind in der Außenwand 16 drei Durchgangsöffnungen vorgesehen. Die Deckenwand des Sterilisierbehälterdeckels 22 sowie der Boden 14 sind demgegenüber frei von Öffnungen.

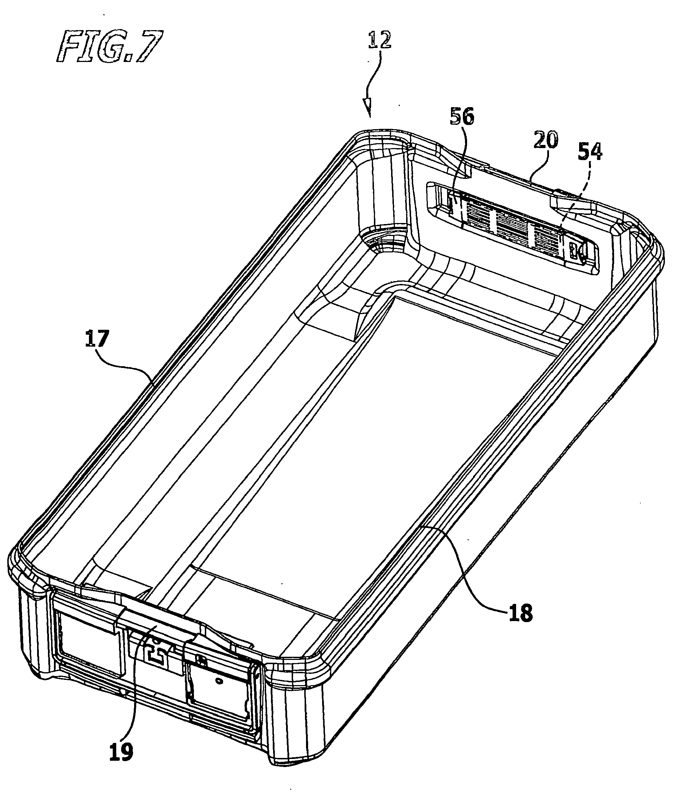

Eine erste Durchgangsöffnung ist, dies ist in der Zeichnung nicht dargestellt, in der Querseitenwand 20 gebildet. Die Durchgangsöffnung wird von einem Filter 54 überdeckt (Figur 7). Der Filter 54 wird mittels eines Filterhalteelementes 56 in Gestalt eines Haltebleches die Durchgangsöffnung überdeckend an der Querseitenwand 20 gehalten. Durch den Filter 54 hindurch erfolgt ein Medienaustausch zwischen der Umgebung und dem Behälterinnenraum 24, sofern eine erste maximale Druckdifferenz zwischen der Umgebung und dem Behälterinnenraum nicht überschritten bzw. eine zweite maximale Druckdifferenz zwischen dem Behälterinnenraum 24 und der Umgebung nicht überschritten wird.

Um in den letztgenannten Fällen einen Medienaustausch zu ermöglichen und eine Beschädigung des Filters 54 zu vermeiden, umfasst der Sterilisierbehälter 10 eine Ventileinrichtung 58. Die Ventileinrichtung 58 dient zum Freigeben und Verschließen von zwei Durchgangsöffnungen 60 und 62, die in der Querseitenwand 19 seitlich nebeneinander und im Abstand zum Boden 14 gebildet sind. Die Durchgangsöffnungen 60 sind vorliegend kreisförmig. Zum Verschließen und Freigeben der Durchgangsöffnungen 60 und 62 umfasst die Ventileinrichtung 58 ein Einlassventil 64 bzw. ein Auslassventil 66.

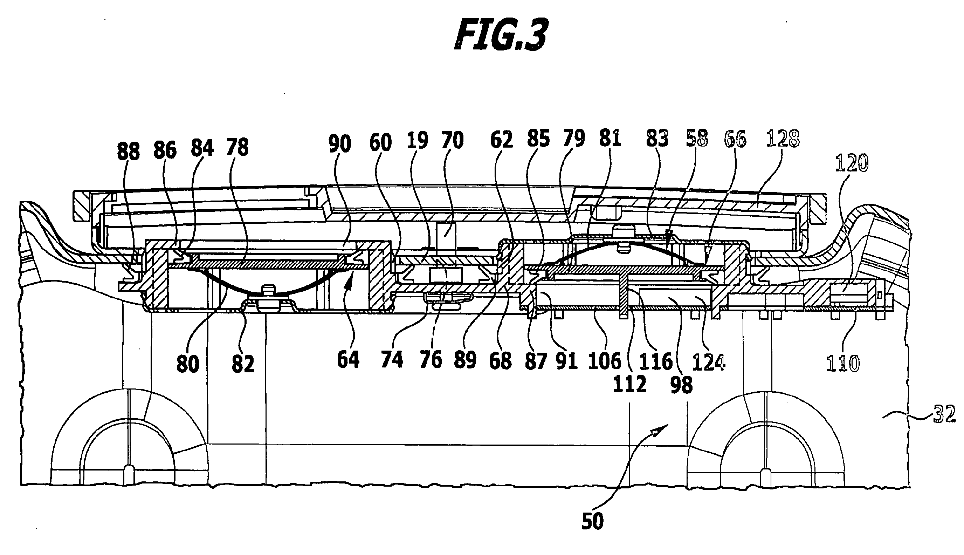

Den Ventilen 64 und 66 ist ein vorliegend einstückiges Ventilhalteteil 68 zugeordnet, an dem die Ventile 64 und 66 gehalten sind. Das Ventilhalteteil 68 ist im Wesentlichen plattenförmig ausgestaltet und mit der Querseitenwand 19 lösbar verbunden. Zu diesem Zweck sind Verbindungselemente in Gestalt von Nieten vorgesehen (ein Niet 70 ist in Figur 3 gezeigt). Der Niet 70 durchgreift eine Durchbrechung 72 im Ventilhalteteil 68, relativ zu dem er mittels eines Klemmelementes 74 kraft- und formschlüssig fixiert ist. Das Klemmelement 74 ist als Klemmschiene ausgestaltet, mittels derer beide Niete mit dem Ventilhalteteil 68 fixiert werden können. Mit dem freien Ende durchgreift der Niet 70 eine Durchbrechung 76 der Querseitenwand 19. Mit dem Rand der Durchbrechung 76 steht der Niet 70 in kraft- und formschlüssigem Eingriff, so dass er dadurch an der Querseitenwand 19 fixiert ist.

Zum Lösen des Ventilhalteteils 68 von der Querseitenwand 19 kann der kraftschlüssige Eingriff der Niete mit dem Klemmelement 74 aufgehoben werden und das Ventilhalteteil 68 mit den Ventilen 64 und 66 von der Sterilisierbehälterwanne 12 gelöst werden. Umgekehrt kann das Ventilhalteteil 68 mit der Sterilisierbehälterwanne 12 verbunden und dabei mit dem Klemmelement 74 an dieser fixiert werden.

Das Einlassventil 64 und das Auslassventil 66 sind druckbetätigte Ventile mit scheibenförmigen Ventilkörpern 78 bzw. 79, die sich mittels elastischer Rückstellelemente 80 bzw. 81 in Gestalt von Bügelfedern an mediendurchlässigen Ventilabdeckungen 82 bzw. 83 abstützen, die mit dem Ventilhalteteil 68 verbunden sind. Mittels Dichtelementen 84 bzw. 85, vorliegend in Gestalt von Lippendichtungen, können die Ventilkörper 78 bzw. 79 an Ventilsitzen 86 bzw. 87 des Einlassventils 64 bzw. des Auslassventils 66 dichtend anliegen. Die Ventilsitze 86 und 87 werden durch das Ventilhalteteil 68 ausgebildet. Über Dichtelemente 88 bzw. 89, vorliegend ebenfalls in Gestalt von Lippendichtungen, die die Ränder der Durchgangsöffnungen 60 und 62 umlaufen, ist das Ventilhalteteil dichtend in die Durchgangsöffnungen 60 und 62 eingesetzt. Dadurch verringert das Ventilhalteteil 68 die Querschnittsfläche der Durchgangsöffnungen 60 und 62. Soweit vorliegend von Freigeben bzw. Verschließen der Durchgangsöffnungen 60 und 62 durch das Einlassventil 62 bzw. das Auslassventil 66 die Rede ist, bezieht sich dies auf Durchgangsöffnungen 90 bzw. 91 im Ventilhalteteil 68, deren Ränder in die Durchgangsöffnungen 60 und 62 eingesetzt sind, so dass die Querschnitte der Durchgangsöffnungen 90 und 91 geringer sind als diejenigen der Durchgangsöffnungen 60 bzw. 62.

Der erfindungsgemäße Sterilisierbehälter 10 umfasst eine Fluidhebeeinrichtung 92, um Fluid aus dem Fluidsammelbereich 50 anzuheben und dem Auslassventil 66 zuzuführen. Fluid, insbesondere während des Sterilisiervorgangs gebildetes Kondensat, kann im Fluidsammelbereich 50, insbesondere in der Vertiefung 32 im Bereich der Querseitenwand 19 und der Längsseitenwand 17 gesammelt werden. Die Fluidhebeeinrichtung 92 stellt eine Fluidverbindung her zwischen dem Fluidsammelbereich 50 und dem Auslassventil 66, um Fluid aus dem Behälterinnenraum 24 zu entfernen.

Die Fluidhebeeinrichtung 92 umfasst zu diesem Zweck einen Fluidkanal 94 mit einer Kanaleintrittsöffnung 96 und einer Kanalaustrittsöffnung 98. Der Fluidkanal 94 weist einen die Kanaleintrittsöffnung 96 bildenden ersten Kanalabschnitt 100 sowie einen die Kanalaustrittsöffnung 98 bildenden zweiten Kanalabschnitt 102 auf.

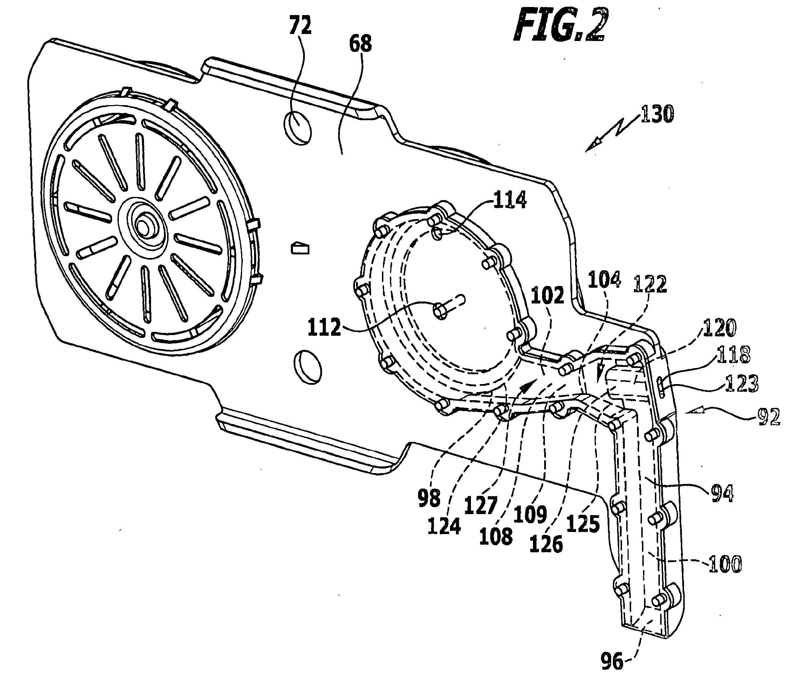

Der erste Kanalabschnitt 100 greift in die Vertiefung 32 am Eckbereich der Querseitenwand 19 mit der Längsseitenwand 17 und damit in den Fluidsammelbereich 50 ein, wobei er die Bodenwand 15 jedoch nicht kontaktiert. Der Abstand des Kanalabschnitts 100 zur Bodenwand beträgt beispielsweise ungefähr 1 bis 5 mm. Relativ zur Aufstellebene 26 ist der erste Kanalabschnitt 100 senkrecht ausgerichtet und erstreckt sich von dieser wegweisend nach oben ungefähr bis zur halben Höhe der Querseitenwand 19. Die Kanaleintrittsöffnung 96 ist auf die Bodenwand 15 gerichtet, worunter verstanden werden kann, dass die der Durchtrittsrichtung von Fluid durch die Kanaleintrittsöffnung 96 entgegengesetzte Richtung auf die Bodenwand 15 weist (Figur 1). Die Eintrittsöffnung 96 weist eine Neigung bezüglich der Bodenwand 15 auf infolge einer Abschrägung des ersten Kanalabschnitts 100 an dem der Bodenwand 15 zugewandten Ende (Figur 2).

Der zweite Kanalabschnitt 102 und der erste Kanalabschnitt 100 sind in einem Winkel relativ zueinander ausgerichtet, der vorliegend kleiner ist als 90°. Eine vom zweiten Kanalabschnitt 102 definierte Richtung ist relativ zur Aufstellebene 26 geneigt, so dass der zweite Kanalabschnitt 102 in Richtung der Aufstellebene 26 abfällt. Die Kanalaustrittsöffnung 98 ist an einer dem Boden 14 zugewandten Seite des Auslassventils 66 angeordnet. Die Kanalaustrittsöffnung 98 ist auf die Aufstromseite des Auslassventils 66 gerichtet, wobei die Durchtrittsrichtung von Fluid durch die Kanalaustrittsöffnung 98 im Wesentlichen parallel zu einer vom scheibenförmigen Ventilkörper 79 definierten Ebene ist.

Der zweite Kanalabschnitt 102 weist eine Kanalverengung 104 auf, im Bereich derer die Querschnittsfläche des Fluidkanals 94 verkleinert ist. In Strömungsrichtung des Fluids durch den Fluidkanal 94 nachgelagert erweitert sich der Querschnitt des Fluidkanals 94 nach der Kanalverengung 104 bis zur Kanalaustrittsöffnung 98 wieder.

Der Fluidkanal 94 wird gebildet durch Kanalwände, die ausgebildet werden vom Ventilhalteteil 68 sowie einer mit diesem verbundenen Abdeckung 106. Im Ventilhalteteil 68 ist zu diesem Zweck eine Nut 108 gebildet, die eine erste Kanalwand 109 des Fluidkanals 94 bildet. Die der Kanalwand 109 gegenüberliegende Kanalwand 110 wird gebildet durch die Abdeckung 106. Die Kanalwände 109 und 110 werden durch seitliche Nutwände der Nut 108 vom Ventilhalteteil 68 gebildet.

Die Abdeckung 106 überdeckt die Nut 108 und die Durchgangsöffnung 91 im Ventilhalteteil 68 und damit das Auslassventil 66 behälterinnenseitig. Der Fluidkanal 94 verläuft aufgrund der vorstehend erwähnten Ausgestaltung zwischen dem Ventilhalteteil 68 und der vorliegend plattenförmigen Abdeckung 106 im Wesentlichen in einer bzw. parallel zu einer vom Ventilhalteteil 68 definierten Ebene.

Aufgrund der starren Ausgestaltung des Ventilhalteteils 68 und der Abdeckung 106 ist der Fluidkanal 94 formstabil. Über das Ventilhalteteil 68 ist der Fluidkanal 94 mit der Querseitenwand 19 lösbar verbunden.

Die Abdeckung 106 weist in ihrem die Durchgangsöffnung 91 überdeckenden Abschnitt zwei Durchbrechungen 112 und 114 auf. In die zentrale Durchbrechung 112 greift ein vorliegend zapfenförmiger Vorsprung 116 am Ventilkörper 79 ein, wenn das Auslassventil 66 geschlossen ist. Der Vorsprung 116 und die Durchbrechung 112 dienen damit als zusammenwirkende Ausrichtelemente zur Ausrichtung des Ventilkörpers 79 relativ zur Abdeckung 106. Die Durchbrechung 114 ist oberhalb der Durchbrechung 112 angeordnet und liegt dem Außenrand des Ventilkörpers 79 behälterinnenseitig gegenüber.

Im Übergangsbereich vom ersten Kanalabschnitt 100 zum zweiten Kanalabschnitt 102 weist der Fluidkanal 94 eine Eintrittsöffnung 118 auf, in Verlängerung der vom zweiten Kanalabschnitt 102 definierten Richtung. Ein hülsenförmiger Einsatz 120, dessen Durchgang mit der Eintrittsöffnung 118 fluchtet, ist im Fluidkanal 94 angeordnet, im Abzweigungsbereich des zweiten Kanalabschnitts 102 vom ersten Kanalabschnitt 100. Bezogen auf die Strömungsrichtung von Fluid durch den Fluidkanal 94 sind der Einsatz 120 und damit die Eintrittsöffnung 118 der Kanalverengung 104 vorgelagert.

Die Fluidhebeeinrichtung 92 umfasst einen in den Fluidkanal 94 integrierten Injektor 122 mit einer Injektoreintrittsöffnung 123, einer Injektoraustrittsöffnung 124 und einer Saugöffnung 125. Die Injektoreintrittsöffnung 123 wird ausgebildet durch die Eintrittsöffnung 118 des Fluidkanals 94, und die Injektoraustrittsöffnung 124 wird gebildet durch die Kanalaustrittsöffnung 98. Die Saugöffnung 125 wird gebildet durch den Fluidkanal 94 in dessen den Einsatz 120 umgebenden Bereich. Der Einsatz 120 hat die Funktion einer Düse 126 des Injektors 122. Der Injektor umfasst ferner einen Diffusor 127, der in den Kanal integriert ist und abschnittsweise vom zweiten Kanalabschnitt 102 gebildet wird. Der Diffusor 127 erstreckt sich von der Kanalverengung 104 bis zur Kanalaustrittsöffnung 98.

Nachfolgend wird auf Zweck und Funktion der Fluidhebeeinrichtung 92 in Kombination mit dem Auslassventil 66 eingegangen. Die Fluidhebeeinrichtung 92 ist vorgesehen, um eine Flüssigkeit, insbesondere Kondensat, am Ende des Sterilisiervorgangs aus dem Behälterinnenraum 24 zu entfernen. Dabei wird der Umgebungsdruck um den Sterilisierbehälter 10 so weit erniedrigt, bis er den Innendruck des Sterilisierbehälters 10 deutlich unterschreitet. Infolge der Druckdifferenz öffnet das druckgesteuerte Auslassventil 66, so dass ein Druckausgleich mit dem Umgebungsdruck erfolgen kann. Gas und Fluid können aus dem Sterilisierbehälter 10 austreten.

Zum Öffnen des Auslassventils 66 erweist es sich als vorteilhaft, dass durch die Durchbrechungen 112 und 114 der Abdeckung 106 und durch die Eintrittsöffnung 118 im Fluidkanal 94 Strömungsverbindungen zwischen dem Auslassventil 66 und dem Behälterinnenraum 24 gebildet sind, bei hinreichend hohem Kondensatpegel in der Vertiefung 32 unter Umgehung des ersten Kanalabschnitts 100. Aufgrund des erhöhten Behälterinnendrucks können sich durch die Eintrittsöffnung 118 und den zweiten Kanalabschnitt 102 sowie durch die Durchbrechungen 112 und 114 hindurch unter Öffnen des Auslassventils 66 Nebenstrompfade am ersten Kanalabschnitt 100 vorbei ausbilden.

Je nach Höhe des Kondensatpegels im Fluidsammelbereich 50 ist es jedoch auch möglich, dass sich eine Gasströmung durch den ersten Kanalabschnitt 100 ausbildet, wenn der Kondensatpegel so niedrig ist, dass der erste Kanalabschnitt 100 mit der Kanaleintrittsöffnung 96 nicht vollständig in Fluid eintaucht.

Öffnet das Auslassventil 66, entsteht durch den Injektor 122 eine Saugströmung, indem Gas durch die Eintrittsöffnung 118 und die Düse 126 sowie den zweiten Kanalabschnitt 102 zum Auslassventil 66 strömt. Infolge der Kanalverengung 104 erfolgt eine Druckminderung an der Saugöffnung 125, so dass sich eine Druckdifferenz im Fluidkanal 94 zwischen der Saugöffnung 125 und dem Druck an der Kanaleintrittsöffnung 96 ausbildet. Dies führt dazu, dass Fluid aus dem Fluidsammelbereich in den Fluidkanal 94 eingesaugt wird. Weiter wird Fluid durch den Fluidkanal 94 angehoben und dem Auslassventil 66 zugeführt, unter andauernder Saugströmung durch den Injektor 122.

Als vorteilhaft erweist es sich dabei, dass der zweite Kanalabschnitt 102 in Richtung auf die Aufstellebene 26 geneigt ist. Dies führt dazu, dass sich die Strömung angehobenen Fluids beruhigt, so dass es nach Verlassen des Fluidkanals 94 nicht sprühstoßartig aus dem Sterilisierbehälter 10 austritt. Zu diesem Zweck ist auch der Diffusor 127 zwischen der Kanalverengung 104 und der Kanalaustrittsöffnung 98 vorgesehen, mit dem eine Drucksteigerung bei gleichzeitiger Beruhigung der Strömung des Fluids sichergestellt wird. Zum Schutz von Versprühen von austretendem Fluid ist ferner ein im Wesentlichen plattenförmiges Abdeckelement 128 an der Außenseite der Querseitenwand 19 gehalten. Das Abdeckelement 128 dient ferner zur Aufnahme und Lagerung eines Behältergriffs des Sterilisierbehälters 10.

Das Anheben von Fluid aus dem Fluidsammelbereich 50 durch den Fluidkanal 94 hindurch unter der Wirkung der Saugströmung durch den Injektor 122 ist auch dann möglich und wirksam, wenn der Fluidpegel so weit abgefallen ist, dass der erste Kanalabschnitt 100 nicht vollständig mit der Kanaleintrittsöffnung 96 in das Fluid eintaucht. Auch bei einer gemischten Kondensat- und Gasströmung durch den ersten Kanalabschnitt 100 hindurch zeigt sich, dass unter der Wirkung der Saugströmung durch den Injektor 122 weiterhin Fluid wirkungsvoll aus dem Fluidsammelbereich 50 angehoben und dem Auslassventil 66 zugeführt werden kann.

Weiter erweist es sich, insbesondere bei hohem, die Kanaleintrittsöffnung 96 übersteigenden Kondensatpegel als vorteilhaft, dass durch die Durchbrechungen 112 und 114 und die Eintrittsöffnung 118 Nebenstrompfade am ersten Kanalabschnitt 100 vorbei vorhanden sind. Dies führt zu einem Druckabbau im Behälterinnenraum 24 über mehr als nur einen Strömungspfad, so dass Fluid nicht schwallartig durch den Fluidkanal 94 abgesaugt und aus dem Sterilisierbehälter 10 entfernt wird. Auch die mechanische Belastung des Sterilisierbehälters 10 kann dadurch verringert werden.

Nach Schließen des Auslassventils 66 im Sterilisierbehälter 10 möglicherweise noch vorhandenes Fluid kann während der Trocknungsphase nach dem eigentlichen Sterilisiervorgang durch die Restwärme speziell der Sterilisierbehälterwanne 12 verdampfen und durch den Filter 54 hindurch aus dem Behälterinnenraum 24 austreten. Hierzu erweist es sich als vorteilhaft, dass der Fluidsammelbereich 50 längs der Querseitenwand 19 verläuft, so dass auch die in der Querseitenwand 19 gespeicherte Restwärme zum Verdampfen von Fluid wirksam ist. Entsprechend verhält es sich mit dem Fluidsammelbereich 52, der längs der Querseitenwand 20 verläuft.

Das Vorsehen der Fluidhebeeinrichtung 92 erweist sich als vorteilhaft, um eine große Menge an Fluid wirksam anzuheben und aus dem Behälterinnenraum 24 zu entfernen, noch bevor die eigentliche Trocknungsphase des Sterilisiervorganges einsetzt. Die Trocknungsphase kann dadurch wesentlich verkürzt werden.

Weiter ist es besonders günstig, dass der Boden 14 frei von jeglichen Durchgangsöffnungen ist. Auf Ventile zum Verschließen des Bodens 14 kann dadurch verzichtet werden, und der Boden 14 bildet eine Sterilbarriere. Die Gefahr eines Eindringens von Keimen durch Durchgangsöffnungen im Boden, wie dies bei Sterilisierbehältern mit Fluidablassventilen im Boden der Fall ist, kann dadurch auch nach dem Ende des Sterilisiervorgangs vermieden werden. Auch eine Beschädigung oder Fehlfunktion eines Fluidablassventils kann verhindert werden.

Das Auslassventil 66 und die Fluidhebeeinrichtung 92 sind vorliegend Bestandteil einer bevorzugten Ausführungsform einer in Figur 2 perspektivisch dargestellten und mit dem Bezugszeichen 130 belegten erfindungsgemäßen chirurgischen Fluidextraktionsvorrichtung, die beim Sterilisierbehälter 10 zum Einsatz kommt. Die Fluidextraktionsvorrichtung 130 umfasst ferner das Ventilhalteteil 68 sowie das Einlassventil 64. Es kann alternativ vorgesehen sein, dass das Einlassventil 64 nicht im Ventilhalteteil 68 gehalten ist und dementsprechend kein Bestandteil der Fluidextraktionsvorrichtung 130 ist. Wie erwähnt, kann die Fluidextraktionsvorrichtung 130 lösbar mit der Querseitenwand 19 verbunden werden, was über eine Montageerleichterung hinaus auch deren Austausch bei Bedarf ermöglicht.

## Patentansprüche

1. Chirurgischer Sterilisierbehälter, umfassend einen Boden (14) und eine Behälterwand (16), wobei am Sterilisierbehälter (10) eine Durchgangsöffnung (62) zum Medienaustausch gebildet ist, sowie umfassend eine Ventileinrichtung (58), die ein Auslassventil (66) zum Freigeben und Verschließen der Durchgangsöffnung (62) aufweist, wobei die Durchgangsöffnung (62) in der Behälterwand (16) gebildet ist und einen Abstand zum Boden (14) aufweist, **dadurch gekennzeichnet, dass** der Sterilisierbehälter (10) eine Fluidhebeeinrichtung (92) umfasst zum Bereitstellen einer Fluidverbindung vom Boden (14) zum Auslassventil (66) und zum Anheben von Fluid vom Boden (14) zum Auslassventil (66), dass die Fluidhebeeinrichtung (92) einen Fluidkanal (94) umfasst zum Bereitstellen der Fluidverbindung, durch den hindurch Fluid vom Boden (14) zum Auslassventil (66) anhebbar ist und der eine Kanaleintrittsöffnung (96) für Fluid umfasst sowie eine im Abstand dazu angeordnete Kanalaustrittsöffnung (98) für Fluid, und dass der Fluidkanal (94) mit einem die Kanaleintrittsöffnung (96) bildenden Ende in eine im Boden (14) gebildete Vertiefung (32) eingreift, die einen Fluidsammelbereich (50) zum Sammeln von Fluid ausbildet.

2. Sterilisierbehälter nach Anspruch 1, **dadurch gekennzeichnet, dass** der Boden (14) frei von Durchgangsöffnungen ist.

3. Sterilisierbehälter nach einem der voranstehenden Ansprüche, **dadurch gekennzeichnet, dass** die Fluidhebeeinrichtung (92) druckbetätigbar ist.

4. Sterilisierbehälter nach einem der voranstehenden Ansprüche, **dadurch gekennzeichnet, dass** die Fluidhebeeinrichtung (92) einen Injektor (122) zum Anheben von Fluid durch den Fluidkanal (96) umfasst und dass der Injektor (122) eine Injektoreintrittsöffnung (123) und eine Injektoraustrittsöffnung (124) aufweist, über die eine Strömungsverbindung von einem vom Sterilisierbehälter (10) definierten Behälterinnenraum (24) zum Auslassventil (66) bereitgestellt wird, sowie eine in Strömungsrichtung zwischen der Injektoreintrittsöffnung (123) und der Injektoraustrittsöffnung (124) angeordnete und vom Fluidkanal (94) ausgebildete Injektorsaugöffnung (125).

5. Sterilisierbehälter nach Anspruch 4, **dadurch gekennzeichnet, dass** der Injektor (122) in den Fluidkanal (94) integriert ist, wobei die Injektoraustrittsöffnung (124) durch die Kanalaustrittsöffnung (98) gebildet ist und/oder die Injektoreintrittsöffnung (123) in einer Kanalwand des Fluidkanals (94) gebildet ist.

6. Sterilisierbehälter nach einem der voranstehenden Ansprüche, **dadurch gekennzeichnet, dass** der Fluidkanal (94) einen ersten, in einer vom Boden (14) weg weisenden Richtung ausgerichteten Kanalabschnitt (100) aufweist, sowie einen, bezogen auf die Strömungsrichtung des vom Boden (14) zum Auslassventil strömenden Fluids, dem ersten Kanalabschnitt (100) nachgelagerten zweiten Kanalabschnitt (102), der einen Winkel mit dem ersten Kanalabschnitt (100) einschließt.

7. Sterilisierbehälter nach Anspruch 6, **dadurch gekennzeichnet, dass** der zweite Kanalabschnitt (102) eine Neigung relativ zu einer vom Sterilisierbehälter (10) definierten Aufstellebene (26) aufweist.

8. Sterilisierbehälter nach einem der voranstehenden Ansprüche, **dadurch gekennzeichnet, dass** der Fluidkanal (94) abschnittsweise von einer Kanalwand (109) begrenzt ist, die von einem Ventilhalteteil (68) gebildet ist, an welchem das Auslassventil (66) gehalten ist und das an der Behälterwand (16) festgelegt ist.

9. Sterilisierbehälter nach einem der voranstehenden Ansprüche, **dadurch gekennzeichnet, dass** die Fluidhebeeinrichtung (92) eine den Fluidkanal (94) abschnittsweise begrenzende Kanalwand (110) umfasst, die eine das Auslassventil (66) behälterinnenseitig überdeckende Abdeckung (106) ausbildet.

10. Sterilisierbehälter nach Anspruch 9, **dadurch gekennzeichnet, dass** in der Abdeckung (106) mindestens eine Öffnung (112, 114) zum Ausbilden einer Strömungsverbindung von einem vom Sterilisierbehälter (10) definierten Behälterinnenraum (24) zum Auslassventil (66) vorgesehen ist.

11. Sterilisierbehälter nach Anspruch 9 oder 10, **dadurch gekennzeichnet, dass** die Abdeckung (106) mit dem Ventilhalteteil (68) verbunden ist und der Fluidkanal (94) zwischen der Abdeckung (106) und dem Ventilhalteteil (68) gebildet ist.

12. Sterilisierbehälter nach einem der voranstehenden Ansprüche, **dadurch gekennzeichnet, dass** der Sterilisierbehälter (10) ein Ventilhalteteil (68) umfasst, an dem das Auslassventil (66) gehalten ist und das an der Behälterwand (19) festgelegt ist.

13. Sterilisierbehälter nach Anspruch 12, **dadurch gekennzeichnet, dass** das Ventilhalteteil (68) in die Durchgangsöffnung (62) eingesetzt ist und einen Ventilsitz (87) des Auslassventils (66) ausbildet.

14. Sterilisierbehälter nach Anspruch 12 oder 13, **dadurch gekennzeichnet, dass** die Ventileinrichtung (58) ein Einlassventil (61) umfasst, das am Ventilhalteteil (68) gehalten ist.

15. Verwendung einer Fluidextraktionsvorrichtung der in einem Sterilisierbehälter nach Anspruch 1, eine Behälterwand (16) und einen Boden (14) aufweist, wobei die Fluidextraktionsvorrichtung (130) zum Extrahieren von Fluid aus dem Sterilisierbehälter (10) eine Ventileinrichtung (58) umfasst, welche ein Auslassventil (66) aufweist, mit dem eine in der Behälterwand (16) des Sterilisierbehälters (10) gebildete Durchgangsöffnung (62) freigebbar und verschließbar ist, und eine Fluidhebeeinrichtung (92) umfasst zum Bereitstellen einer Fluidverbindung vom Boden (14) zum Auslassventil (66) und zum Anheben von Fluid vom Boden (14) zum Auslassventil (66), wobei die Fluidhebeeinrichtung (92) einen Fluidkanal (94) umfasst zum Bereitstellen der Fluidverbindung, durch den hindurch Fluid vom Boden (14) zum Auslassventil (66) anhebbar ist und der eine Kanaleintrittsöffnung (96) für Fluid umfasst sowie eine im Abstand dazu angeordnete Kanalaustrittsöffnung (98) für Fluid, die in eine im Boden (14) gebildete Vertiefung (32) eingreifen kann, welche Vertiefung (32) einen Fluidsammelbereich (50) zum Sammeln von Fluid ausbildet.

## Claims

1. Surgical sterilizing container, comprising a bottom (14) and a container wall (16), a through-opening (62) for the exchange of media being formed on the sterilizing container (10), and comprising a valve device (58) having an outlet valve (66) for opening and closing the through-opening (62), the through-opening (62) being formed in the container wall (16) and being at a distance from the bottom (14), **characterized in that** the sterilizing container (10) comprises a fluid lifting device (92) for providing a fluid connection from the bottom (14) to the outlet valve (66) and for lifting fluid from the bottom (14) to the outlet valve (66), **in that** the fluid lifting device (92) comprises a fluid channel (94) for providing the fluid connection, through which fluid is liftable from the bottom (14) to the outlet valve (66) and which has a channel inlet opening (96) for fluid and a channel outlet opening (98) for fluid arranged at a distance from the latter, and **in that** the fluid channel (94) with an end forming the channel inlet opening (96) engages in a depression (32) which is formed in the bottom (14) and forms a fluid collection area (50) for collecting fluid.

2. Sterilizing container in accordance with claim 1, **characterized in that** the bottom (14) is free of through-openings.

3. Sterilizing container in accordance with any one of the preceding claims, **characterized in that** the fluid lifting device (92) is pressure-actuatable.

4. Sterilizing container in accordance with any one of the preceding claims, **characterized in that** the fluid lifting device (92) comprises an injector (122) for lifting fluid through the fluid channel (96), and **in that** the injector (122) comprises an injector inlet opening (123) and an injector outlet opening (124), via which a flow connection is provided from a container interior (24) defined by the sterilizing container (10) to the outlet valve (66), and also an injector suction opening (125) arranged in the direction of flow between the injector inlet opening (123) and the injector outlet opening (124) and formed by the fluid channel (94).

5. Sterilizing container in accordance with claim 4, **characterized in that** the injector (122) is integrated in the fluid channel (94), the injector outlet opening (124) being formed by the channel outlet opening (98) and/or the injector inlet opening (123) being formed in a channel wall of the fluid channel (94).

6. Sterilizing container in accordance with any one of the preceding claims, **characterized in that** the fluid channel (94) has a first channel section (100) aligned in a direction facing away from the bottom (14), and, in relation to the direction of flow of the fluid flowing from the bottom (14) to the outlet valve, downstream of the first channel section (100), a second channel section (102) which includes an angle with the first channel section (100).

7. Sterilizing container in accordance with claim 6, **characterized in that** the second channel section (102) has an inclination relative to a setdown plane (26) defined by the sterilizing container (10).

8. Sterilizing container in accordance with any one of the preceding claims, **characterized in that** a section of the fluid channel (94) is delimited by a channel wall (109) formed by a valve holder (68) on which the outlet valve (66) is held and which is fixed to the container wall (16).

9. Sterilizing container in accordance with any one of the preceding claims, **characterized in that** the fluid lifting device (92) comprises a channel wall (110) which delimits a section of the fluid channel (94) and forms a cover (106) covering the outlet valve (66) on the inner side of the container.

10. Sterilizing container in accordance with claim 9, **characterized in that** at least one opening (112, 114) for forming a flow connection from a container interior (24) defined by the sterilizing container (10) to the outlet valve (66) is provided in the cover (106).

11. Sterilizing container in accordance with any one of claims 9 or 10, **characterized in that** the cover (106) is connected to the valve holder (68), and the fluid channel (94) is formed between the cover (106) and the valve holder (68).

12. Sterilizing container in accordance with any one of the preceding claims, **characterized in that** the sterilizing container (10) comprises a valve holder (68) on which the outlet valve (66) is held and which is fixed to the container wall (19).

13. Sterilizing container in accordance with claim 12, **characterized in that** the valve holder (68) is inserted in the through-opening (62) and forms a valve seat (87) of the outlet valve (66).

14. Sterilizing container in accordance with any one of claims 12 or 13, **characterized in that** the valve device (58) comprises an inlet valve (61) which is held on the valve holder (68).

15. Use of a fluid extraction device in a sterilizing container in accordance with claim 1 which has a container wall (16) and a bottom (14), the fluid extraction device (130) comprising, for extracting fluid from the sterilizing container (10), a valve device (58) which has an outlet valve (66) with which a through-opening (62) formed in the container wall (16) of the sterilizing container (10) is openable and closable, and comprising a fluid lifting device (92) for providing a fluid connection from the bottom (14) to the outlet valve (66) and for lifting fluid from the bottom (14) to the outlet valve (66), the fluid lifting device (92) comprising a fluid channel (94) for providing the fluid connection, through which fluid is liftable from the bottom (14) to the outlet valve (66) and which has a channel inlet opening (96) for fluid and a channel outlet opening (98) for fluid arranged at a distance from the latter, which channel outlet opening (98) can engage in a depression (32) being formed in the bottom (14), which depression (32) forms a fluid collection area (50) for collecting fluid.

## Revendications

1. Récipient de stérilisation chirurgical, comprenant un fond (14) et une paroi de récipient (16), une ouverture de passage (62) pour l'échange du milieu étant formée sur le récipient de stérilisation (10), le récipient comportant également un dispositif de valve (58), qui comprend une valve de sortie (66) pour dégager et fermer l'ouverture de passage (62), l'ouverture de passage (62) étant formée dans la paroi de récipient (16) et étant située à distance du fond (14),
**caractérisé en ce que** le récipient de stérilisation (10) comporte un dispositif de levage de fluide (92) pour réaliser une liaison fluidique du fond (14) à la valve de sortie (66) et assurer le levage de fluide du fond (14) à la valve de sortie (66), **en ce que** le dispositif de levage de fluide (92) comprend un canal de fluide (94) pour réaliser la liaison fluidique, et à travers lequel du fluide peut être soulevé du fond (14) jusqu'à la valve de sortie (66), et qui comporte une ouverture d'entrée de canal (96) pour du fluide ainsi que, agencée à distance de celle-ci, une ouverture de sortie de canal (98) pour du fluide, et **en ce que** le canal de fluide (94) s'engage, avec une extrémité formant l'ouverture d'entrée de canal (96), dans un creux (32), qui est réalisé dans le fond (14) et forme une zone de collecte de fluide (50) destinée à collecter du fluide.

2. Récipient de stérilisation selon la revendication 1, **caractérisé en ce que** le fond (14) est exempt d'ouvertures de passage.

3. Récipient de stérilisation selon l'une des revendications précédentes, **caractérisé en ce que** le dispositif de levage de fluide (92) peut être actionné par la pression.

4. Récipient de stérilisation selon l'une des revendications précédentes, **caractérisé en ce que** le dispositif de levage de fluide (92) comprend un injecteur (122) pour faire monter du fluide à travers le canal de fluide (96), et **en ce que** l'injecteur (122) comprend une ouverture d'entrée d'injecteur (123) et une ouverture de sortie d'injecteur (124), par l'intermédiaire desquelles est établie une liaison d'écoulement à partir d'un espace intérieur de récipient (24) défini par le récipient de stérilisation (10) vers la valve de sortie (66), ainsi qu'une ouverture d'aspiration d'injecteur (125), qui est agencée, en se référant à la direction d'écoulement, entre l'ouverture d'entrée d'injecteur (123) et l'ouverture de sortie d'injecteur (124), et qui est formée par le canal de fluide (94).

5. Récipient de stérilisation selon la revendication 4, **caractérisé en ce que** l'injecteur (122) est intégré au canal de fluide (94), l'ouverture de sortie d'injecteur (124) étant formée par l'ouverture de sortie de canal (98) et/ou l'ouverture d'entrée d'injecteur (123) étant formée dans une paroi de canal du canal de fluide (94).

6. Récipient de stérilisation selon l'une des revendications précédentes, **caractérisé en ce que** le canal de fluide (94) présente un premier tronçon de canal (100) orienté dans une direction s'éloignant du fond (14), ainsi qu'un deuxième tronçon de canal (102), qui, en se référant à la direction d'écoulement du fluide s'écoulant du fond (14) vers la valve de sortie, est situé en aval du premier tronçon de canal (100), et forme un angle avec le premier tronçon de canal (100).

7. Récipient de stérilisation selon la revendication 6, **caractérisé en ce que** le deuxième tronçon de canal (102) présente une inclinaison par rapport à un plan de pose (26), qui est défini par le récipient de stérilisation (10).

8. Récipient de stérilisation selon l'une des revendications précédentes, **caractérisé en ce que** le canal de fluide (94) est délimité partiellement par une paroi de canal (109) formée par une partie de support de valve (68), sur laquelle est maintenue la valve de sortie (66), et qui est fixée à la paroi de récipient (16).

9. Récipient de stérilisation selon l'une des revendications précédentes, **caractérisé en ce que** le dispositif de levage de fluide (92) comprend une paroi de canal (110) délimitant partiellement le canal de fluide (94) et formant un élément de recouvrement (106), qui recouvre la valve de sortie (66) du côté intérieur du récipient.

10. Récipient de stérilisation selon la revendication 9, **caractérisé en ce que** dans l'élément de recouvrement (106) est prévue au moins une ouverture (112, 114) pour réaliser une liaison d'écoulement d'un espace intérieur de récipient (24) défini par le récipient (10), à la valve de sortie (66).

11. Récipient de stérilisation selon la revendication 9 ou la revendication 10, **caractérisé en ce que** l'élément de recouvrement (106) est relié à la partie de support de valve (68), et le canal de fluide (94) est formé entre l'élément de recouvrement (106) et la partie de support de valve (68).

12. Récipient de stérilisation selon l'une des revendications précédentes, **caractérisé en ce que** le récipient de stérilisation (10) comprend une partie de support de valve (68) sur laquelle est fixée la valve de sortie (66) et qui est fixée à la paroi de récipient (19).

13. Récipient de stérilisation selon la revendication 12, **caractérisé en ce que** la partie de support de valve (68) est insérée dans l'ouverture de passage (62) et forme un siège de valve (87) de la valve de sortie (66).

14. Récipient de stérilisation selon la revendication 12 ou la revendication 13, **caractérisé en ce que** le dispositif de valve (58) comprend une valve d'entrée (61), qui est maintenue sur la partie de support de valve (68).

15. Utilisation d'un dispositif d'extraction de fluide dans un récipient de stérilisation selon la revendication 1, qui comporte une paroi de récipient (16) et un fond (14), le dispositif d'extraction de fluide (130) comprenant, en vue d'extraire du fluide du récipient de stérilisation (10), un dispositif de valve (58), qui comporte une valve de sortie (66) à l'aide de laquelle une ouverture de passage (62) formée dans la paroi de récipient (16) du récipient de stérilisation (10), peut être dégagée ou fermée, et comprenant également un dispositif de levage de fluide (92) pour réaliser une liaison fluidique du fond (14) à la valve de sortie (66) et assurer le levage de fluide du fond (14) à la valve de sortie (66), le dispositif de levage de fluide (92) comprenant un canal de fluide (94) pour réaliser la liaison fluidique, et à travers lequel du fluide peut être soulevé du fond (14) jusqu'à la valve de sortie (66), et qui comporte une ouverture d'entrée de canal (96) pour du fluide ainsi que, agencée à distance de celle-ci, une ouverture de sortie de canal (98) pour du fluide, qui peut s'engager dans un creux (32) formé dans le fond (14) et ledit creux (32) formant une zone de collecte de fluide (50) destinée à collecter du fluide.
